# EUROPÄISCHE PATENTANMELDUNG

(11) **EP 2 873 374 A1**
(43) Veröffentlichungstag der Anmeldung: **20.05.2015**
(21) Anmeldenummer: 14003576.7
(22) Anmeldetag: 20.10.2014
(51) Int. Cl.: A61B 17/00, A61B 19/04, A61M 5/32, A61F 5/058

(54) **Fingerstütze zum Anlegen an einen Finger**

(30) Priorität: 19.11.2013 DE 102013019383; 27.06.2014 DE 102014009333
(71) Anmelder: Thurn, Martin, 97421 Schweinfurt (DE)
(72) Erfinder: Thurn, Martin, 97421 Schweinfurt (DE)
(74) Vertreter: Gosdin, Michael

(57) **Zusammenfassung**

Die Erfindung betrifft eine Fingerstütze (1) zum Anlegen an einen Finger (2), insbesondere zum zumindest zeitweisen Stützen des Fingers (2) während eines Dialysevorgangs oder nach einem Dialysevorgang, wobei die Fingerstütze (1) ein Stützelement (3) umfasst, das einen ersten Stützabschnitt (3`) aufweist, der sich entlang der Unterseite des Endgliedes (2') des Fingers (2) erstreckt, das einen zweiten Stützabschnitt (3 ") aufweist, der sich entlang der Unterseite des sich an das Endglied (2') anschließenden Glieds (2") des Fingers (2) erstreckt, und einen dritten Stützabschnitt (3'") aufweist, der sich entlang der Unterseite des weiter anschließenden Glieds (2"') des Fingers (2) erstreckt, wobei der erste Stützabschnitt (3'), der zweiten Stützabschnitt (3") und der dritte Stützabschnitt (3"') einstückig ausgebildet sind. Um ein effektives Abdrücken eines durchstochenen Gewebebereichs zu erreichen und ansonsten einen effektiven Schutz gegen Stiche zu gewährleisten, sieht die Erfindung vor, dass der erste Stützabschnitt (3') zumindest abschnittsweise kappenartig (4) ausgebildet ist, so dass er das Ende des in den ersten Stützabschnitt (3') eingelegten Endglieds (2') des Fingers (2) zumindest teilweise umgibt, wobei in der Seitenansicht der Fingerstütze (1) zwischen dem zweiten und dem dritten Stützabschnitt (3", 3'") ein Winkel (α) eingeschlossen ist, der zwischen 10° und 60° liegt.

## Beschreibung

Die Erfindung betrifft eine Fingerstütze zum Anlegen an einen Finger, insbesondere zum zumindest zeitweisen Stützen des Fingers während eines Dialysevorgangs oder nach einem Dialysevorgang, wobei die Fingerstütze ein Stützelement umfasst, das einen ersten Stützabschnitt aufweist, der sich entlang der Unterseite des Endgliedes des Fingers erstreckt, das einen zweiten Stützabschnitt aufweist, der sich entlang der Unterseite des sich an das Endglied anschließenden Glieds des Fingers erstreckt, und einen dritten Stützabschnitt aufweist, der sich entlang der Unterseite des weiter anschließenden Glieds des Fingers erstreckt, wobei der erste Stützabschnitt, der zweiten Stützabschnitt und der dritte Stützabschnitt einstückig ausgebildet sind.

Eine Fingerstütze dieser Art ist aus der DE 85 30 483 U1 bekannt, wobei die Fingerstützte dafür dient, einen erkrankten Finger bis zu seiner Heilung ruhig zu stellen.

Bei der Dialyse werden sog. Shunts (oder auch arteriovenöse Fisteln genannt) eingesetzt. Es handelt sich dabei um Kurzschlussverbindungen zum Blutübertritt zwischen normalerweise getrennten Gefäßen (Arterien und Venen).

Beim betroffenen Dialysepatienten wird künstlich ein Shunt angelegt, um ein großvolumiges Gefäß zur Verfügung zu haben, mit dem man eine Hämodialyse durchführen kann.

Der bevorzugte Ort für einen Dialyseshunt ist die Verbindung zwischen der Arteria radialis und der Vena cephalica am Unterarm. Weitere Möglichkeiten für einen Dialyseshunt am Oberarm sind die Verbindungen der mobilisierten und unter die Haut verlagerten Vena basilica mit der Arteria brachialis (sog. Basilica-Shunt) oder zwischen Vena cephalica und Arteria brachialis (sog. Cephalica-Shunt).

Hierbei kommen zwei Nadeln zum Einsatz, die in die jeweiligen Blutgefäße einstechen. Nach dem Dialysevorgang werden die Nadeln gezogen und die Durchstichstelle abgedrückt, um Blutaustritt zu verhindern und ein schnelles Verschließen der durchstochenen Stelle zu fördern. Hierfür ist ein hinreichender Druck auf die Durchstichstelle aufzubauen.

Das Abdrücken des Shuntlochs nach dem Nadelziehen ist relativ kräftezehrend, da über einen hinreichenden Zeitraum der Druck aufrecht erhalten werden muss. Dies gilt schon für den Krankenpfleger, aber noch mehr für den Patienten, wenn dieser die Aufgabe des Abdrückens übernimmt.

Ein weiterer Aspekt beim Shuntziehen ist ein Stichschutz gegen die nach oben gelangende zweite Shunt-Nadel. Problematisch ist hier insbesondere, dass eine hohe Gefahr besteht, dass beim Ziehen des Shunts die zweite Nadel den Krankenpfleger sticht. Hierdurch besteht die Gefahr, dass es hier zu Infektionen kommt (insbesondere bezüglich Hepatitis C und AIDS).

Der Erfindung liegt daher die Aufgabe zugrunde, eine Fingerstütze der eingangs genannten Art vorzuschlagen, mit der ein effizientes und kräftesparendes Abdrücken des Shunt-Lochs in der Haut möglich wird. Weiterhin soll die Fingerstütze einen effektiven Schutz gegen unbeabsichtigtes Stechen mit einer Shunt-Nadel gewährleisten. Da die in Rede stehende Fingerstütze in großen Stückzahlen benötigt wird, soll schließlich eine sehr kostengünstige Herstellung möglich sein.

Die Lösung dieser Aufgabe durch die Erfindung ist dadurch gekennzeichnet, dass der erste Stützabschnitt zumindest abschnittsweise kappenartig ausgebildet ist, so dass er das Ende des in den ersten Stützabschnitt eingelegten Endglieds des Fingers zumindest teilweise umgibt, wobei in der Seitenansicht der Fingerstütze zwischen dem zweiten und dem dritten Stützabschnitt ein Winkel eingeschlossen ist, der zwischen 10° und 60° liegt.

Dabei ist bevorzugt vorgesehen, dass der erste Stützabschnitt das Ende des in ihn eingelegten Endglieds des Fingers so umgibt, dass bei bestimmungsgemäßem Gebrauch der Fingerstütze die gesamte Fingerkuppe des Endglieds des Fingers bis oberhalb des Fingernagels umgeben ist.

Die Stützabschnitte weisen bevorzugt an ihrer dem Finger zugewandten Seite eine konkave Ausformung auf. Sie sind in diesem Falle also der Form der Finger bzw. dessen Glieder angepasst.

An mindestens einem der Stützabschnitte kann eine Halterung angeordnet sein, die bei bestimmungsgemäßem Gebrauch das gestützte Fingerglied zumindest abschnittsweise zirkulär umgibt. Die Halterung umgibt dabei bevorzugt das gestützte Fingerglied vollständig zirkulär. Die Halterung kann dabei aus dem Material des Stützabschnitts bestehen. Möglich ist es allerdings auch, dass das Fingerglied von der Halterung nicht vollständig zirkulär umgeben wird, sondern einen Schlitz aufweist, was die Anpassbarkeit an unterschiedliche Fingergrößen erleichtert.

Es ist aber auch möglich, dass die Halterung bandförmig ausgebildet ist und einen Klettverschluss umfasst.

Die Fingerstütze kann gemäß einer Weiterbildung an ihrem von der kappenartigen Ausformung entfernten Ende eine bogenförmige Ausformung aufweisen.

Bevorzugt besteht die Fingerstütze aus Kunststoff. Sie ist dabei bevorzugt per Spritzgießen hergestellt.

Sehr vorteilhaft ist es, dass der zweite und dritte Stützabschnitt nicht gerade, d. h. fluchtend zueinander ausgerichtet sind, sondern zueinander den genannten Winkel einschließen.

Das Abdrücken des Shuntlochs kann dann mit wesentlich geringerer Anstrengung erfolgen, als wenn der Finger gestreckt wäre. Der Winkel liegt erfindungsgemäß in einem Bereich zwischen 10° und 60°, wobei ein bevorzugter Bereich zwischen 15° und 45° liegt.

Das Abdrücken des Shuntlochs wird ferner dadurch erleichtert und ist für den Patienten angenehmer, wenn die kappenartige Ausformung nicht ausschließlich aus hartem Kunststoffmaterial besteht, sondern entweder an der Innenseite zum abdrückenden Finger hin oder an der Außenseite zur Abdrückstelle hin - oder sowohl innen als auch außen - eine Schicht aus weicherem Material vorhanden ist, d. h. aus einem Material, das weicher ist als das härtere Kunststoffmaterial des Stützelements.

Hierbei kann vorgesehen werden, dass die Fingerstütze im 2-Komponenten-Spritzgießverfahren hergestellt wird, wobei dann die zweite, weichere Komponente in situ, d. h. direkt im Spritzgießwerkzeug angespritzt wird. Es kann aber auch vorgesehen werden, dass eine Lage aus weicherem Material auf das härtere Material des Stützabschnitts aufgebracht, beispielsweise aufgeklebt, wird.

Die vorgeschlagene Fingerstütze stellt also eine Kombination eines Schutzes des abdrückenden Fingers vor Stichverletzungen und eine schienenförmige Unterstützung beim Abdrücken des Shuntlochs zur Verfügung. Das Infektionsrisiko des Pflegepersonals kann so signifikant reduziert werden.

Die Fingerstütze wird genutzt, um nach dem Nadelziehen am Shuntarm den Abdrückfinger zu stützen und eine vorteilhafte Position zu geben, in der das Abdrücken erleichtert ist.

Die Fingerstütze ist der Anatomie des Fingers angepasst, wobei insbesondere durch eine geschlitzte Halterung eine Anpassbarkeit an verschiedene Fingergrößen gegeben ist. Durch die günstige ergonomische Gestaltung der Fingerschiene kann auch ein langes Abdrücken des Shuntlochs ohne Verkrampfungen erfolgen.

In der Zeichnung ist ein Ausführungsbeispiel der Erfindung dargestellt. Es zeigen:
- Fig. 1: die Seitenansicht eines Fingers, mit dem ein Shuntloch abgedrückt werden soll,
- Fig. 2: die Seitenansicht einer Hand, an der eine erfindungsgemäße Fingerstütze angelegt ist,
- Fig. 3: die Seitenansicht der Hand mit schematischer Darstellung eines Arms, aus dem ein Shunt gezogen werden muss, und
- Fig. 4: einen Schnitt durch die Fingerstütze, wobei nur der Bereich des Endglieds des stützenden Fingers dargestellt ist.

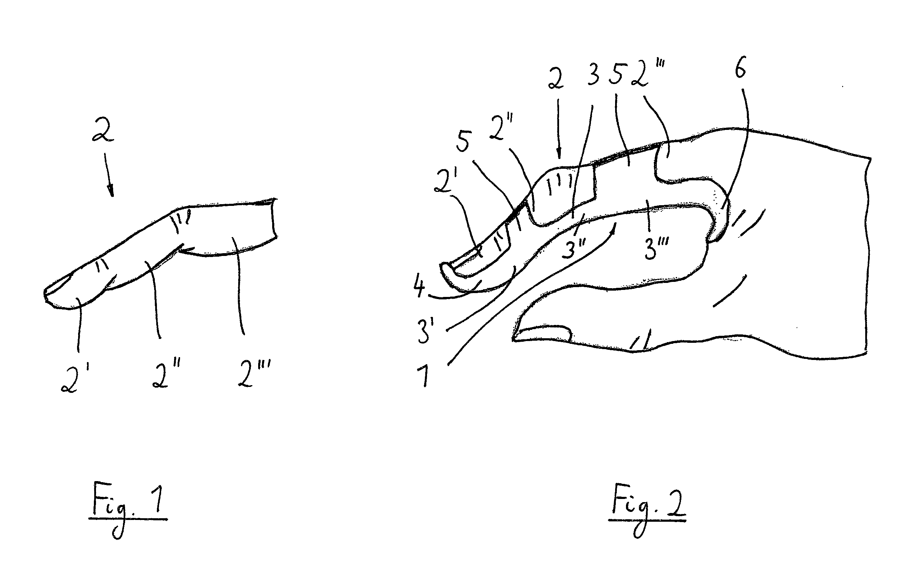

In Fig. 1 ist ein Finger 2 zu sehen, an den die nachfolgend beschriebene Fingerstütze 1 angelegt werden kann, um das effektive Abdrücken eines Shunt-Lochs nach der Dialyse und dem Ziehen des Shunts bewerkstelligen zu können. Der Finger 2 hat ein Endglied 2', ein sich an dieses anschließende Glied 2" und ein weiteres Fingerglied 2"'. Im Falle, dass der Daumen für das Abdrücken des Shuntlochs eingesetzt wird, liegen entsprechend nicht drei, sondern nur zwei Fingerglieder vor.

In Fig. 2 ist eine Hand eines Krankenpflegers skizziert, wobei an den Finger 2 die erfindungsgemäße Fingerstütze 1 angelegt wurde.

Die Fingerstütze 1 besteht vorliegend aus einem Stützelement 3, das aus drei Bereichen besteht, nämlich aus einem ersten Stützabschnitt 3', einem sich an diesen anschließenden zweiten Stützabschnitt 3" und einen sich wiederum an diesen anschließenden dritten Stützabschnitt 3"'. Alle drei Stützabschnitte 3', 3", 3'" sind einstückig aus einem Kunststoffmaterial gefertigt, wobei sich beispielsweise Polyamid eignet. Die drei Stützabschnitte 3', 3", 3'" korrespondieren betreffend ihre Ausdehnung in Längsrichtung der jeweiligen Fingerglieder dem Endglied 2', dem sich anschließenden Glied 2" und dem weiteren Fingerglied 3'".

Dabei ist der erste Stützabschnitt 3' kappenartig ausgeformt; diese kappenartige Ausformung ist mit der Bezugsziffer 4 versehen. Die kappenartige Ausformung 4 ist dabei in zwei zueinander senkrecht stehenden Richtungen jeweils konvex ausgeformt, so dass sich ein Kugelabschnitt bzw. ein Abschnitt eines Ellipsoids ergibt, der die Fingerkuppe des Endglieds 2' des Fingers 2 umgibt. Wie sich aus Fig. 2 ergibt, ist diese Einfassung der Fingerkuppe jedoch nicht vollständig. Wie in der Figur gesehen werden kann, wird vielmehr nur etwa die untere Hälfte des Endglieds 2' von der kappenartigen Ausformung 4 eingefasst; diese schließt noch den Fingernagel ein, lässt den weiteren, oberen Bereich des Endglieds 2' indes frei.

An der Fingerstütze 1 sind zwei Halterungen 5 angeformt. Diese sind vorliegend aus dem Material der Fingerstütze 1 direkt ausgebildet und können durch den Spritzgießvorgang, durch den die Fingerstütze 1 bevorzugt gefertigt wird, in einfacher Weise hergestellt werden. Die Halterungen sind im Ausführungsbeispiel ringförmig ausgeführt. Sie können an der Oberseite auch einen in Richtung der Längsachse der Fingerglieder verlaufenden Schlitz aufweisen, um die Anpassbarkeit an verschiedene Fingerdicken zu ermöglichen.

Nicht dargestellt ist die Möglichkeit, die Halterungen bandförmig auszubilden und per Klettverschluss am entsprechenden Fingerglied lösbar festzulegen.

In Fig. 2 ist auch noch zu erkennen, dass die Fingerstütze 1 in ihrem Endbereich, der von der kappenartigen Ausformung 4 entfernt ist, bogenförmig ausgebildet ist. Aus Fig. 2 ist gut zu erkennen, dass damit eine gute Anlage im Endbereich des Fingers 2 an der Stelle gegeben ist, die in den Daumen übergeht. Diese bogenförmige Ausformung 6 unterstützt das Aufbringen einer hinreichenden Abdruckkraft auf das Shunt-Loch während der hierfür benötigten Zeit.

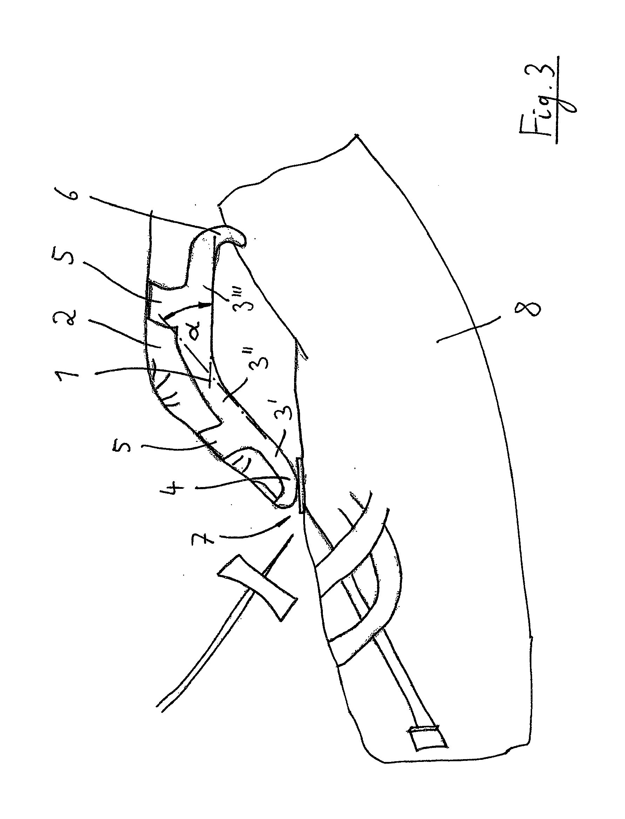

Der Abdrückvorgang des Shunt-Lochs ist schematisch in Fig. 3 skizziert. Hier ist der Shuntarm 8 angedeutet, in dem der Shunt platziert ist, was nur sehr schematisch angedeutet ist. Der Finger 2, an dem die Fingerstütze 1 angelegt ist, kann ohne große Kraftanstrengung auf die Abdrückstelle 7 gepresst werden, um die Blutung zu stoppen.

Wie zu erkennen ist, ist durch die Fingerstütze 1 eine stützende Funktion für den Finger 2 gewährleistet, auch wenn die Nadel durchsticht. Da insofern ein Wegziehen des gestützten Fingers infolge eines unbeabsichtigten Stichs verhindert wird, ist eine stetige Abdrückfunktion gewährleistet.

Damit ist ein effizientes Abdrücken ohne übermäßigen Kraftaufwand sichergestellt.

Um das Abdrücken des Shuntlochs einfach und wenig kräftezehrend zu gestalten, ist in Fig. 3 noch eine hervorzuhebende Ausgestaltung dargestellt. Wie nämlich gesehen werden kann, sind hier die beiden in Fingerlängsrichtung aufeinander folgenden Stützabschnitte 3" und 3"' nicht gerade, sondern unter einem Winkel α zueinander ausgerichtet. Der Winkel beträgt im Ausführungsbeispiel ca. 40°. Durch diese winkelige Anordnung der beiden genannten Stützabschnitte kann das Abdrücken des Shuntlochs jetzt mit geringem Kraftaufwand über die benötigte Zeit erfolgen; bei gestrecktem Finger wäre das nicht so kräftesparend möglich.

Die Fingerstütze 1 ist bevorzugt als einteiliges Spritzgießformteil ausgeführt. Die Dicke (gemessen senkrecht auf die Hautoberfläche des gestützten bzw. geschützten Fingers) liegt bevorzugt zwischen 0,5 mm und 3 mm, was auch vom verwendeten Material abhängt. Bevorzugt wird ein hartes Plastikmaterial, beispielsweise Polyamid.

Durch die ergonomisch der gestützten Fingeroberfläche angepasste Form der Fingerstütze (d. h. durch die konkave Ausgestaltung an der dem Finger zugewandten Oberfläche der Fingerstütze sowie durch die kappenartige Ausformung im Bereich des Endglieds) wird der Fingerstütze 1 eine gegen Biegung stabile Form verliehen. Dies begünstigt, dass das Abdrücken des Shuntlochs mit geringem Kraftaufwand und ohne hohe Belastung für das Pflegepersonal bzw. den Patienten selber möglich ist.

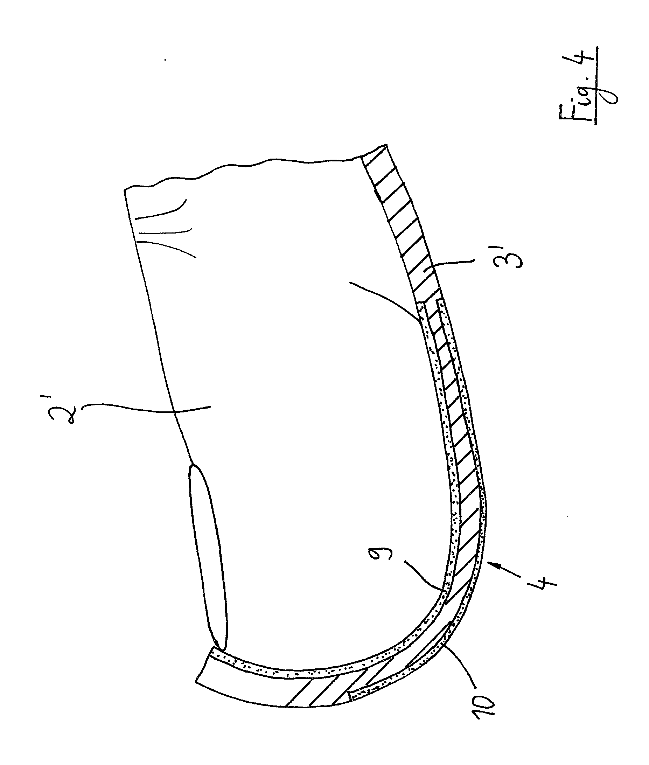

In Fig. 4 ist ein weiteres Detail einer bevorzugten Ausführungsform der erfindungsgemäßen Fingerstütze 1 dargestellt.

Sowohl für den abdrückenden Finger 2 als auch für den Shuntarm 8 ist das Abdrücken sehr viel angenehmer, wenn kein direkter Kontakt mit dem relativ harten Kunststoffmaterial besteht, aus dem die Fingerstütze 1 hergestellt ist. Daher ist - wie in Fig. 4 gesehen werden kann - vorgesehen, dass die kappenartige Ausformung 4 im Ausführungsbeispiel sowohl an der Innenseite als auch an der Außenseite mit einer Schicht eines weicheren Materials versehen ist. Hierbei handelt es sich bevorzugt um ein geschäumtes Kunststoffmaterial, wobei beispielsweise Polyurethan in Frage kommt. Im Ausführungsbeispiel ist die Fingerstütze 1 im 2-K-Verfahren spritzgegossen, so dass die beiden Schichten 9 und 10 aus weicherem Material direkt während des Spritzgießvorgangs angespritzt wurden.

Die Shore-Härte des weicheren Materials, d. h. der Schichten 9 und 10, ist dabei bevorzugt um mindestens 20 Shore-Härtegrade (A) kleiner als die des härteren Materials des Stützelements 3. Das Material des Stützelements 3 besteht also aus einem Material mit einem ersten Shore-Härtegrad (z. B. aus kompaktem Polyamid), das weichere Material der Schichten 9 bzw. 10 hat einen zweiten Shore-Härtegrad, der um mindestens 20 Shore-Härtegrade (A) niedriger liegt (z. B. geschäumtes Polyurethan).

Die praktische Durchführung der Dialyse wird durch den Einsatz der vorgeschlagenen Fingerstütze vorteilhaft vereinfacht.

### Bezugszeichenliste:

- 1: Fingerstütze
- 2: Finger (Daumen)
- 2': Endglied
- 2'': sich anschließendes Glied
- 2''': weiteres Fingerglied
- 3: Stützelement
- 3': erster Stützabschnitt
- 3'': zweiter Stützabschnitt
- 3''': dritter Stützabschnitt
- 4: kappenartige Ausformung
- 5: Halterung
- 6: bogenförmige Ausformung
- 7: Abdrückstelle
- 8: Shuntarm
- 9: Schicht aus weicherem Material
- 10: Schicht aus weicherem Material

- α: Winkel

## Patentansprüche

1. Fingerstütze (1) zum Anlegen an einen Finger (2), insbesondere zum zumindest zeitweisen Stützen des Fingers (2) während eines Dialysevorgangs oder nach einem Dialysevorgang, wobei die Fingerstütze (1) ein Stützelement (3) umfasst, das einen ersten Stützabschnitt (3') aufweist, der sich entlang der Unterseite des Endgliedes (2') des Fingers (2) erstreckt, das einen zweiten Stützabschnitt (3 ") aufweist, der sich entlang der Unterseite des sich an das Endglied (2') anschließenden Glieds (2") des Fingers (2) erstreckt, und einen dritten Stützabschnitt (3"') aufweist, der sich entlang der Unterseite des weiter anschließenden Glieds (2"') des Fingers (2) erstreckt, wobei der erste Stützabschnitt (3'), der zweiten Stützabschnitt (3 ") und der dritte Stützabschnitt (3"') einstückig ausgebildet sind,
**dadurch gekennzeichnet,**
**dass** der erste Stützabschnitt (3') zumindest abschnittsweise kappenartig (4) ausgebildet ist, so dass er das Ende des in den ersten Stützabschnitt (3') eingelegten Endglieds (2') des Fingers (2) zumindest teilweise umgibt, wobei in der Seitenansicht der Fingerstütze (1) zwischen dem zweiten und dem dritten Stützabschnitt (3", 3"') ein Winkel (α) eingeschlossen ist, der zwischen 10° und 60° liegt.

2. Fingerstütze nach Anspruch 1, **dadurch gekennzeichnet, dass** der erste Stützabschnitt (3') das Ende des in ihn eingelegten Endglieds (2') des Fingers (2) so umgibt, dass bei bestimmungsgemäßem Gebrauch der Fingerstütze die gesamte Fingerkuppe des Endglieds (2') des Fingers (2) bis oberhalb des Fingernagels umgeben ist.

3. Fingerstütze nach Anspruch 1 oder 2, **dadurch gekennzeichnet, dass** die Stützabschnitte (3', 3", 3"') an ihrer dem Finger (2) zugewandten Seite eine konkave Ausformung aufweisen.

4. Fingerstütze nach einem der Ansprüche 1 bis 3, **dadurch gekennzeichnet, dass** an mindestens einem der Stützabschnitte (3', 3", 3"') eine Halterung (5) angeordnet ist, die bei bestimmungsgemäßem Gebrauch das gestützte Fingerglied (2', 2", 2"') zumindest abschnittsweise zirkulär umgibt.

5. Fingerstütze nach Anspruch 4, **dadurch gekennzeichnet, dass** die Halterung (5) das gestützte Fingerglied (2', 2", 2'") vollständig zirkulär umgibt.

6. Fingerstütze nach Anspruch 4 oder 5, **dadurch gekennzeichnet, dass** die Halterung (5) aus dem Material des Stützabschnitts (3) besteht.

7. Fingerstütze nach Anspruch 4 oder 5, **dadurch gekennzeichnet, dass** die Halterung (5) bandförmig ausgebildet ist und einen Klettverschluss umfasst.

8. Fingerstütze nach einem der Ansprüche 1 bis 7, **dadurch gekennzeichnet, dass** sie an ihrem von der kappenartige Ausformung (4) entfernten Ende eine bogenförmige Ausformung (6) aufweist.

9. Fingerstütze nach einem der Ansprüche 1 bis 8, **dadurch gekennzeichnet, dass** sie aus Kunststoff besteht.
